(19) Europäisches Patentamt / European Patent Office / Office européen des brevets

(11) EP 1 656 955 B1

(12) **EUROPEAN PATENT SPECIFICATION**

(45) Date of publication and mention of the grant of the patent:
**29.08.2007 Bulletin 2007/35**

(21) Application number: **04745633.0**

(22) Date of filing: **07.06.2004**

(51) Int Cl.:
***A61L 12/14*** *(2006.01)* ***A01N 25/22*** *(2006.01)*
***A01N 33/12*** *(2006.01)* ***A01N 47/44*** *(2006.01)*
***C11D 7/26*** *(2006.01)* ***C11D 7/32*** *(2006.01)*

(86) International application number:
**PCT/JP2004/007923**

(87) International publication number:
**WO 2005/018693 (03.03.2005 Gazette 2005/09)**

(54) **LIQUID PREPARATION FOR CONTACT LENS**

FLÜSSIGE ZUBEREITUNG FÜR KONTAKTLINSEN

PREPARATION LIQUIDE POUR LENTILLES DE CONTACT

(84) Designated Contracting States:
**AT BE BG CH CY CZ DE DK EE ES FI FR GB GR HU IE IT LI LU MC NL PL PT RO SE SI SK TR**

(30) Priority: **20.08.2003 JP 2003295863**

(43) Date of publication of application:
**17.05.2006 Bulletin 2006/20**

(73) Proprietor: **Menicon Co., Ltd.**
**Nagoya-shi, Aichi 460-0006 (JP)**

(72) Inventor: **MORI, Osamu,**
**c/o MENICON CO., LTD.**
**Kasugai-shi,**
**Aichi 487 0032 (JP)**

(74) Representative: **Paget, Hugh Charles Edward et al**
**Mewburn Ellis LLP**
**York House**
**23 Kingsway**
**London WC2B 6HP (GB)**

(56) References cited:
**EP-A- 1 050 304**
**WO-A-98/25649**
**WO-A-98/50084**
**JP-A- 10 137 327**
**JP-A- 2001 242 428**
**JP-A- 2002 136 578**
**JP-A- 2003 116 972**
**JP-A- 2003 160 482**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 656 955 B1

## Description

TECHNICAL FIELD

The present invention relates in general to a liquid preparation for a contact lens, and more particularly to a liquid preparation, which is advantageously used, at a time of sterilizing, cleaning, and storing the contact lens, as a contact lens disinfecting solution, a contact lens disinfecting and cleaning solution, a contact lens disinfecting and storing solution, and a contact lens disinfecting, cleaning, and storing solution.

BACKGROUND ART

Generally, contact lenses are classified into non-water-contained contact lenses and water-contained contact lenses, hard contact lenses and soft contact lenses, or nonionic contact lenses and ionic contact lenses. During a long period of use of the contact lenses, microorganisms such as bacteria and fungi tend to adhere to and proliferate on the surfaces of the contact lenses while the contact lenses are stored after they have been removed from the eyes. In view of the above, the contact lenses generally need to be sterilized before they are worn on the eyes. In particular, it is especially important to sterilize the soft contact lenses since the microorganisms are likely to proliferate on the surfaces of the soft contact lenses easier than on the hard contact lenses, increasing a risk of causing the infectious diseases.

For this reason, there have been conventionally used liquid preparations for contact lens, in which predetermined disinfectants (preservatives) are included, for thereby executing the sterilizing treatments. As the disinfectants to be added to the liquid preparations for contact lens, there are widely used, for instance, biguanide compounds whose typical example is polyhexamethylene biguanide (PHMB) and quaternary ammonium compounds whose typical examples are benzalkonium chloride and Polyquaternium, because these disinfectants have especially effective disinfecting properties, compared with the other disinfectants.

However, for assuring a practical requirement in view of the disinfection (preservation), it is necessary to increase the amount of the disinfectants to be included in the liquid preparation, even if the above-mentioned effective disinfectants are used. The disinfectant if used in high concentration, is toxic to the eyes, and the disinfectant is easily adsorbed on the contact lens, so that there is an anxiety of causing eye troubles such as eye irritation, giving rise to a problem of insufficient safety. In view of this, various studies have been made to provide a liquid preparation for contact lens capable of exhibiting a higher disinfecting effect while reducing the required amount of the disinfectant.

For example, in JP-A-10-108899 (Patent document 1), there is proposed a liquid preparation for contact lens, which includes, in addition to 0.1 ppm to 10 ppm of polyhexamethylene biguanide, a nonionic isotonic agent within a ratio to provide a certain extent of an osmotic pressure, which extent is similar to that of a predetermined concentration of sodium chloride. Besides, in JP-A-11-249087 (Patent document 2), there is proposed a liquid preparation for contact lens, which includes a nonionic isotonic agent and/or an amino acid, in addition to a predetermined polyquaternium. It is true that disinfecting effect of the liquid preparation for contact lens can be improved by adopting this constitution of the liquid preparation. However, there is an inherent problem that, depending on the kinds of the nonionic isotonic agent and/or the amino acid to be used, the contact lens, especially a soft contact lens, is swelled or shrunk, so that the size of the contact lens is changed, whereby the specification of the contact lens is changed.

For this reason, in order to restrict the change of the size of the contact lens within an extent that does not substantially obstruct the wearing of the contact lens, JP-A-2001-242428 (Patent document 3) and JP-A-2002-136578 (Patent document 4) explain that it is useful to further add an amino-acid salt, in particular a sodium salt of an amino acid, to the liquid preparation for contact lens, in addition to the above-mentioned components.

The inventors of the present invention, together with other inventors, have previously proposed, in JP-A-2003-160482 (Patent document 5), a liquid preparation for contact lens, which includes: a germicidal biguanide or a germicidal quaternary ammonium salt; a predetermined amino acid (salt); and a predetermined acidic compound having a carboxyl group or a phosphoric acid group, wherein concentration of sodium chloride is 0 to 0.2 % by weight. There is disclosed that, by using the liquid preparation for contact lens, adsorption of the disinfectant on the contact lens is restricted. Owing to this, there is highly assured a safety to the eyes, and prevented the swelling and the shrinkage of the contact lens, whereby the problem of the change of the size of the contact lens can be advantageously solved.

However, further studies regarding the liquid preparation for contact lens by the inventors of the present invention newly revealed that there may be caused a problem of precipitation or deposition of crystals and powdery substances on the contact lens, depending on the combination of the components to be used. In detail, if the contact lens, especially a soft contact lens, is immerged in the liquid preparation, which is obtained by combining PHMB, arginine, and glycolic acid or asparatic acid, for a long period of time, or the contact lens is repeatedly immerged in the liquid preparation, white crystals or white powdery substances, which are assumed to be derived from components of the liquid preparation, may be deposited on the surfaces of the contact lens in the dot-like form. If the components of the liquid preparation are deposited on the contact lens as described above, the field of view of the contact lens is deteriorated, which adversely

affect the optical characteristics of the lens. In addition, the deposits such as the crystals and the powdery substances cause problems of irritating the eyes, easily adhering the deposits to the lens, and so on. Therefore, there is required to further improve the liquid preparation for contact lens proposed by the inventors of the present invention, from the point of view of preventing the deposition of the components of the liquid preparation.

There can be listed the followings, as the prior art documents related to the present invention.

Patent document 1: JP-A-10-108899
Patent document 2: JP-A-11-249087
Patent document 3: JP-A-2000-242428
Patent document 4: JP-A-2002-136578
Patent document 5: JP-A-2003-160482
Patent document 6: JP-A-10-137327
Patent document 7: JP-A-11-52308
Patent document 8: JP-A-2000-513001
Patent document 9: JP-A-57-132115

DISCLOSURE OF THE INVENTION

PROBLEM TO BE SOLVED BY THE INVENTION

The present invention was developed in the light of the background art situations described above. A problem to be solved by the invention is to provide a liquid preparation for a contact lens wherein: the liquid preparation for contact lens assures an excellent disinfecting effect and a sufficiently high degree of safety to the eyes of the lens wearer; the liquid preparation for contact lens does not adversely affect the contact lens, such as a change of the size of the contact lens; and the liquid preparation for contact lens advantageously restrains a generation of deposits on the lens.

MEANS FOR SOLVING THE PROBLEM

The inventors of the present invention have made an extensive research in an effort to solve the above-indicated problem, and found the followings. By adding glycolic acid and/or asparatic acid, which are/is acidic compound(s), and 2-amino - 2 methyl - 1, 3 - propanediol (salt) to a liquid preparation for contact lens including a germicidal biguanide and/or a germicidal quaternary ammonium salt, so as to fulfill a predetermined ratio of these components: an excellent disinfecting effect and safety are assured; the change of the lens size is advantageously restricted; and the generation of deposits on the lens can be effectively prevented.

The above-mentioned problem of the present invention may be solved according to one form of the present invention, which provides a liquid preparation for a contact lens comprising: (A) at least one disinfectant selected from the group consisting of germicidal biguanides and germicidal quaternary ammonium salts; (B) glycolic acid and/or asparatic acid; and (C) 2-amino-2-methyl-1, 3-propanediol or a salt thereof, wherein molar ratio of components of the B and C is 1:20 to 1.3:1.

In a second form of the liquid preparation for contact lens according to the present invention, concentration of sodium chloride in the liquid preparation is adjusted to be 0 to 0.2 w/w%.

In a third form of the present invention, component of the A is included in the liquid preparation in a concentration of 0.1 to 500 ppm.

In a fourth form of the present invention, component of the B is included in the liquid preparation in a concentration of 0.01 to 5 w/w%.

In a fifth form of the liquid preparation for contact lens according to the invention, a neutral amino acid is further included in the liquid preparation.

In a sixth preferred form of the present invention, the neutral amino acid is included in the liquid preparation in a concentration of 0.1 to 4 w/w%.

In a seventh form of the liquid preparation according to the present invention, the neutral amino acid is glycine.

In an eighth desirable form of the present invention, propylene glycol is further included in the liquid preparation.

In a ninth form of the present invention, the propylene glycol is included in the liquid preparation in a concentration of 0.1 to 1w/w %.

In a tenth form of the liquid preparation for a contact lens according to the present invention, at least one of a surfactant and a chelating agent is further included in the liquid preparation.

In an eleventh form of the present invention, the contact lens to be treated with the preparation is a soft contact lens.

## ADVANTAGEOUS EFFECT OF THE INVENTION

In the above-mentioned first form according to the present invention, there are/is used glycolic acid and/or asparatic acid (component B), which generates organic anion by ionizing in an aqueous medium, in addition to the germicidal biguanide and/or the germicidal quaternary ammonium salt (component A). Owing to this, adsorption of the disinfectant on the contact lens is advantageously restricted, and the liquid preparation excels in the safety to the eyes.

Besides, in the first form, 2-amino-2-methyl-1, 3-propanediol or the salt thereof (component C) is further added to the liquid preparation in addition to the above-mentioned component A and component B, wherein the component C is added to the liquid preparation, in an amount to satisfy a predetermined molar ratio of the components B and C. Owing to this, the disinfecting effect of the disinfectant is advantageously realized, whereby an excellent disinfecting effect can be obtained. In addition, owing to the combined use of the components B and C, the change of the size of the contact lens is advantageously restricted, and the generation of the deposits on the contact lens derived from the liquid preparation is also effectively prevented.

In detail, the above-mentioned component A is existed in the aqueous medium in the form of a cation, so that the component A is easy to be attached to or adsorbed on the contact lens, especially an ionic contact lens having a minus charge. However, as the component B, which is ionized in the aqueous medium and generates an organic anion, is added to the liquid preparation, the adsorption of the component A on the contact lens is restricted, whereby the safety to the eyes is advantageously enhanced. However, the above-mentioned organic anion (component B) has a tendency to influence on the cation (component A), so that the disinfecting effect of the component A is disturbed. Meanwhile, in the present invention, the component C, which is ionized in the aqueous medium and generates the organic cation, is further added to the liquid preparation, so as to satisfy the predetermined ratio of the component B and the component C. Owing to this, the obstruction to the disinfection caused by the component B is advantageously reduced, whereby the disinfecting effect of the component A can be effectively realized. If the component B or the component C is independently added to the liquid preparation, thus obtained liquid preparation has tendency to cause shrinkage or swelling of the lens. Meanwhile, in the present invention, the component B and the component C are combined together and used in the predetermined molar ratio, so that the change of the size of the lens is effectively prevented, and an excellent compatibility with the lens can be realized. Moreover, even if the contact lens is immersed in the liquid preparation for contact lens including the above-mentioned components A to C for a long period of time, or the contact lens is repeatedly immerged in the liquid preparation, the components originated from the liquid preparation are not deposited on the surfaces of the contact lens.

In the second form of the liquid preparation for a contact lens of the present invention, the disinfecting effect of the disinfectant is effectively exhibited, so that the excellent disinfecting effect can be advantageously realized.

Besides, according to the third form of the present invention, content of the disinfectant is extremely small. Even if the content of the disinfectant is kept to a small amount as described above, an intended disinfecting effect can be advantageously realized, and is remarkably excels in the safety to the eyes.

In addition, according to the above-mentioned fourth form of the liquid preparation for a contact lens of the present invention, the component B as the essential component is added to the liquid preparation, in the predetermined concentration, so that there can be further advantageously realized the above-mentioned effects of the present invention, e.g., the effect of solving the problems such as the generation of the deposits.

Moreover, in the fifth to seventh forms of the present invention, the above-mentioned effects owing to the present invention can be further advantageously exhibited.

In addition, according to the eighth and ninth forms of the present invention, lipophilicity of the liquid preparation for a contact lens is improved, so that occurrences of eye irritation are more advantageously restricted, and more enhanced comfort can be obtained. Moreover, it is also possible to lower the cost of the liquid preparation.

In addition, according to the tenth form of the liquid preparation for a contact lens of the present invention, further effects according to the additional components are added to the liquid preparation.

Moreover, according to the eleventh form of the present invention, a soft contact lens is treated with the liquid preparation according to the present invention. As described above, in the present invention, the above-mentioned effects are advantageously exhibited to the soft contact lens as well.

## BEST MODE FOR CARRYING OUT THE INVENTION

The liquid preparation for a contact lens according to the present invention is constituted by a water-based aqueous medium containing a predetermined disinfectant or a preservative (component A), to which there are added: glycolic acid and/or asparatic acid (component B); and 2-amino-2-methyl-1, 3-propanediol (AMPD) or a salt thereof (component C), wherein the components B and C are combined with each other to fulfill a predetermined ratio of these components.

It is desirable that the above-mentioned disinfectant (component A) excels in its disinfecting effect and conformity

with the contact lens, eyes, etc., and is still more desirable that the disinfectant is not likely to cause troubles, such as allergy As the disinfectant, there are used at least one of, or any combination of conventionally known various germicidal biguanides and germicidal quaternary ammonium salts. In particular, the germicidal biguanides are especially advantageously used. This is because the disinfecting effect of the germicidal biguanides is not likely to be obstructed by other liquid components, e.g., component B, so that even a small amount of the germicidal biguanides can realize an excellent disinfecting effect, compared with the germicidal quaternary ammonium salts.

Examples of the germicidal biguanides include polyhexamethylene biguanide (PHMB) and biguanide polymer, which is represented by the following formula (I).

[Formula 1]

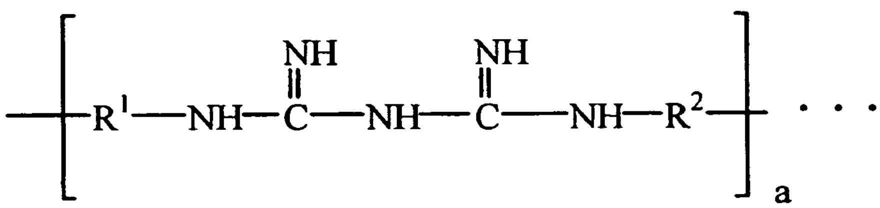

· · · (I)

wherein, a represents an integer of not smaller than 1; and each of $R^1$ and $R^2$ independently represents a divalent group represented by CnHmOp, wherein n=1 ~24, m=2~48, and p=0~ 11.

Examples of the germicidal quaternary ammonium salts include: tetraalkyl ammonium salts such as alkyltrimethylammonium chlorides; alkyl ammonium salt such as trialkylbenzyl ammonium salts such as octadecyldimethylbenzylammonium chloride; quaternary salts of alkylhydroxy alkylimidazoline whose typical example is hydroxyethyl alkylimidazoline chloride; alkylisoquinolinium salts whose typical example is alkylisoquinolinium bromide; alkyl pyridinium salts; and cationic surfactants such as amideamines. In addition to the above, there may also be used polymeric quaternary ammonium compounds represented by the following formulas (II) to (IV), a condensation product of diamines and a dihalogen compound, as disclosed in the Japanese Patent No. 2550036, and a polycationic compounds as disclosed in JP-A-4-231054, JP-A-8-512145, and JP-A-11-249087, cationic cellulose polymer, such as Polyquaternium —4 and Polyquaternium —10, and benzalkonium halide.

[Formula 2]

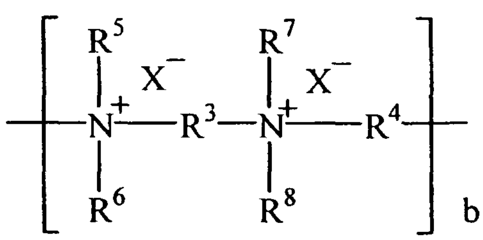

· · · (II)

wherein, b represents an integer of not smaller than 1; $X^-$ is a monovalent anion such as $Cl^-$; each of $R^3$ and $R^4$ independently represents a divalent group represented by CnHmOp, wherein n=1~24, m=2~48, and p=0~11; and each of $R^5$, $R^6$, $R^7$ and $R^8$ independently represents a monovalent group represented by CqHrOs, wherein q=1~4, r=2~9, and s=0~1.

[Formula 3]

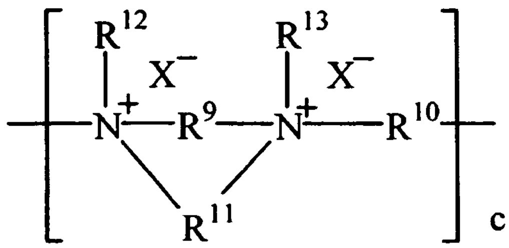

· · · (III)

wherein, c represents an integer of not smaller than 1; $X^-$ is a monovalent anion such as $Cl^-$; each of $R^9$ and $R^{10}$ independently represents a divalent group represented by CnHmOp, wherein n=1~24, m=2~48, and p=0~11; $R^{11}$ represents a monovalent group represented by CtHuOv, wherein t=1~4, u=2~9, and v=0 ~ 1; and each of $R^{12}$ and $R^{13}$ independently represents a monovalent group represented by CqHrOs, wherein q=1~4, r=2 ~9, and s=0~1.

[Formula 4]

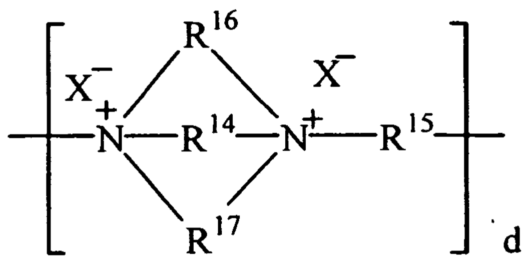

· · · (IV)

wherein, d represents an integer of not smaller than 1; $X^-$ is a monovalent anion such as Cl ; each of $R^{14}$ and $R^{15}$ independently represents a divalent group represented by CnHmOp, wherein n=1~24, m=2~48, and p=0~11; and each of $R^{16}$ and $R^{17}$ independently represents a divalent group represented by CtHuOv, wherein t=1~4, u=2~9, and v=0~1.

The content of the above-mentioned component A is not particularly limited. Generally, a sufficient disinfecting or preservative effect can be obtained by using the component A within a range of about 0.1 to about 1000 ppm. In the present invention, within the above-mentioned range of the concentration, there can be advantageously adopted a low concentration, i.e., 0.1 to 500 ppm, more preferably 0.1 to 200 ppm. In the present invention, there can be obtained an advantageous disinfecting effect, even if the concentration of the component A is very low. If the content of the component A is smaller than the above-mentioned concentration range, there cannot be obtained a sufficient disinfecting (preservative) effect. On the other hand, if the content of the component A exceeds the above-mentioned upper limit of the concentration range, there is an anxiety of causing problems with regard to safety, such as: causing an adverse effect to the eyes, etc.; and accelerating the adsorption of the component A on the surfaces of the contact lens.

Meanwhile, in the liquid preparation for a contact lens according to the present invention, one of, or both of glycolic acid and asparatic acid are included as the component B. In these two acid compounds, glycolic acid is more preferably used, because glycolic acid does not lower the disinfecting effect of the component A, as much as asparatic acid does. A part of, or the entire component B is ionized in the aqueous medium to generate the organic anion. Owing to this, the component A, which is existed as the cation in the aqueous medium, is effectively restrained from adsorbing on the contact lens. Accordingly, there can be advantageously prevented occurrences of eye troubles, such as inflammation and full-staining of the cornea, wherein the entire surface of the cornea is stained by fluorescein during the corneal staining test, whereby safety to the eyes is highly advantageously assured.

The amount of the component B to be included is suitably determined, so as to advantageously restrict the adsorption of the component A on the contact lens. It is generally desirable that the component B is included within a range of 0.01 to 5w/w % (percent by weight), and preferably 0.05 to 1 w/w%. This is because, if the amount of the component B to be included in the liquid preparation is excessively small, there cannot be expected the effect of restricting the adsorption of the component A on the contact lens. On the other hand, if the amount of the component B is excessively large, the contact lens is shrunk and the size of the contact lens is changed, so that there are caused problems in wearing the contact lens.

As aforementioned, by using the above-mentioned component B, the attaching of the component A to the contact lens is restricted, and there is realized an excellent safety to the eyes. However, a mere combination of the component A and the component B tends to result in: shrinking the contact lens; and reducing the disinfecting effect by disturbing the disinfecting effect of the component A, which generates the cation in the aqueous medium. For this reason, in the present invention, component C, which will be described later, is included in the liquid preparation for contact lens, as one of essential components.

In other words, in the liquid preparation for a contact lens in accordance with the present invention, 2-amino-2 - methyl-1, 3-propanediol (AMPD) or the salt thereof is included as the component C. As the salt of AMPD, there may be used hydrochlorides, for instance.

If the component C is added to the liquid preparation, a part of, or all of, the component C is ionized in the aqueous medium and an organic cation is generated. This organic cation works on the above-mentioned organic anion of the component B, whereby the deterioration of the disinfecting effect and the shrinkage of the contact lens caused by the component B are advantageously restricted. Therefore, the disinfecting effect of the component A can be advantageously exhibited, and the change of the size of the contact lens is effectively prevented.

There is assumed that the prevention of the change of the size of the contact lens is owing to the following effects. That is, the liquid preparation for contact lens, which includes the component B without including the component C tends to shrink the contact lens immersed in the liquid preparation, i.e., the liquid preparation tends to change the size of the lens to a smaller size. On the other hand, the liquid preparation for a contact lens, which includes the component C without including the component B tends to swell the contact lens immersed in the liquid preparation, i.e., the liquid preparation tends to change the size of the lens to a larger size. For this reason, there is assumed that, if the components B and C are used together, the shrinkage and the swelling of the contact lens are set off, so that the change of the lens size is hardly occurred.

The amount of the above-mentioned component C to be included in the liquid preparation is suitably determined, according to the amount of the component B to be included in the liquid preparation. In particular, the component C is included in an amount to have the molar ratio of the component B and the component C to be 1:20 to 1.3:1. If the amount of the component C to be included in the liquid preparation exceeds 20 mols per 1 mol of the component B, the adsorption of the component A on the contact lens cannot be effectively prevented. If the amount of the component C to be included in the liquid preparation is less than 1 mol per 1.3 mols of the component B, the disinfecting effect is deteriorated, and the deposits are easily generated. Within the above-mentioned range, desirable molar ratio of the component B and the component C is about 1:15 to about 1.2:1, and preferably about 1:14 to about 1:1.

In the liquid preparation for a contact lens according to the present invention, there is especially adopted, as the component B, glycolic acid and/or asparatic acid among the acid compounds which generate the organic anion, while there is adopted, as the component C, AMPD or a salt thereof among components which generate the organic cation. Owing to this, the disinfecting effect of the component A (disinfectant) is advantageously exhibited, whereby the sufficient disinfecting effect is assured even if the concentration of the component A is very low. Moreover, the change of the size of the contact lens is prevented, whereby the liquid preparation has an excellent compatibility with the lens. In addition, the specific component B and the specific component C are used in combination in the predetermined ratio, so that the generation of the deposits on the contact lens is effectively prevented, when the contact lens is treated with the liquid preparation for contact lens. In short, even if the contact lens is immersed in the liquid preparation for contact lens which includes the above-mentioned components A to C for a long period of time, or the contact lens is repeatedly immersed in the liquid preparation, the crystals or the powdery substances of the components of the liquid preparation are hardly deposited on the surfaces of the contact lens. Owing to this, the field of view of the contact lens is preferably maintained.

The above-mentioned components B and C increase the osmotic pressure of the liquid preparation by being added to the liquid preparation, so that these components also have an effect as an isotonic agent. For this reason, the components B and C are respectively added to and included in the liquid preparation, within a quantitative range, which does not result in exceeding the intended osmotic pressure.

Besides, it is also desirable that the liquid preparation for a contact lens according to the present invention does not include sodium chloride, which is conventionally used as the isotonic agent to adjust the osmotic pressure. If the liquid preparation includes sodium chloride, it is needed to restrict the concentration of the sodium chloride in the liquid preparation to not higher than 0.2 w/w %, and preferably not higher than 0.1 w/w %. In other words, it is desirable that the content of sodium chloride included in the liquid preparation is 0 to 0.2 w/w %, and preferably 0 to 0.1 w/w %. If the content of sodium chloride exceeds 0.2 w/w %, the disinfecting effect of the component A is extremely lowered, so that the intended disinfecting effect cannot be obtained.

The osmotic pressure of the liquid preparation for contact lens is generally within a range of about 250 to about 400 mOsm/kg, which is substantially equal to the physiological osmotic pressure. In the present invention, the concentrations of the components of the liquid preparation are suitably determined, so as to adjust the osmotic pressure to be within the above-mentioned range.

In addition to the above-mentioned components B and C, which function as the isotonic agents, other isotonic agents, in particular, a neutral amino acid (component D) and/or an nonionic isotonic agent (component E) can be added and included in the liquid preparation for contact lens according to the present invention. By further adding these isotonic agents, the deposition of the crystals and powdery substances on the contact lens is further effectively restricted, and the disinfecting effect of the component A is more effectively exhibited. It is needless to mention that these isotonic agents are used within a quantitative range, so as not to exceed the intended value of the osmotic pressure of the liquid preparation.

In detail, as the above-mentioned neutral amino acid, there can be used, for example, glycine, alanine, taurine, ε -aminocaproic acid, etc., and any one of, or any combination of these neutral amino acids can be used. Among them, glycine is especially preferably adopted. This is because, if glycine is used in the liquid preparation: the generation of the deposits of the contact lens is further effectively restricted; the disinfecting effect is enhanced; and the compatibility of the liquid preparation with the contact lens is improved.

If the neutral amino acid is added to the liquid preparation, it is desirable that the amount of the neutral amino acid is 0.1 to 4 w/w % of the liquid preparation. This is because, if the amount of the neutral amino acid to be added is less than 0.1 w/w %, it is difficult to obtain the effect owing to the addition of the neutral amino acid (isotonic effect). On the other hand, if the neutral amino acid to be added is more than 4 w/w %, there is an anxiety of the deposition of the crystals and powdery substances on the contact lens.

Meanwhile, as the above-mentioned nonionic isotonic agent, there can be used, for instance, propylene glycol, glycerin, sugars, etc., and any one of, or any combination of these nonionic isotonic agents can be used. Among them, there is especially preferably adopted propylene glycol. This is because propylene glycol can further effectively restrict the generation of the deposition on the contact lens, and can advantageously lower the occurrences of eye irritation by increasing the viscosity of the liquid preparation.

Where the nonionic isotonic agent is added to the liquid preparation, it is desirable that the amount of the nonionic isotonic agent to be included in the liquid preparation is 0.1 to 1 w/w %. If the amount of the nonionic isotonic agent to be included is less than 0.1 w/w %, it is difficult to obtain the isotonic effect and the effect of reducing the eye irritation owing to the nonionic isotonic agent. On the other hand, if the amount of the nonionic isotonic agent to be included in the liquid preparation is more than 1 w/w %, there is an anxiety that the compatibility of the liquid preparation with the contact lens is adversely affected.

In addition, the liquid preparation for a contact lens according to the present invention may further include, as needed, various known additives as used in the conventional liquid preparations for contact lens, such as a surfactant, a chelating agent, a pH adjusting agent, a buffer, and a thickener. Any one of, or any combination of these additives may be suitably selected and included in the liquid preparation of the present invention. As the additives, there can be adopted any conventionally known additives, as long as they assure a high degree of safety to the living body without giving an adverse influence on the configuration and the physical properties of the contact lens. These additives are added to the liquid preparation within a range of suitable concentration, so as not to adversely influence the functions and effects of the present invention.

The present ophthalmic composition may further include a known surfactant as a cleaning agent, in order to advantageously exhibit a removal effect (cleaning effect) with respect to deposits such as eye lipid.

Examples of the surfactant include: polyglycerin fatty acid ester, polyoxyethylene alkylether, polyoxyethylne-polyoxypropylene block copolymer, polyoxyethylene - polyoxypropylene ethylene diamine, polyoxyethylene sorbitan fatty acid ester, condensation products of polyoxyethylene alkylphenyl ether and formaldehyde, polyoxyethylene hardened castor oil, polyoxyethylene alkylphenyl ether, polyoxyethylene glycerin fatty acid ester, polyoxyethylene sorbitol fatty acid ester, polyoxyethylene castor oil, polyoxyethylene sterol, polyoxyethylene hydrogenated sterol, polyoxyethylene fatty acid ester, polyoxyethylene-polyoxypropylere alkyl ether, polyoxyethylene lanoline alcohol, polyoxyethylene alkyl amine, polyoxyethylene alkyl amide, polyoxyethylene alkyl etherphosphoric acid, and polysorbate, and any one of , or any combination of these surfactants can be used.

The surfactant is advantageously used, generally in a concentration within a range of about 0.001 to about 5 w/w %, preferably about 0.005 to about 2 w/w %, and more preferably about 0.01 to about 1 w/w %. If the amount of the surfactant to be added to the liquid preparation is too small, the cleaning effect will not be sufficient. On the other hand, if the amount of the surfactant to be added to the liquid preparation is too large, there cannot be expected further improvement of the cleaning effect, and instead of this, this would be a cause of eye irritation.

In order to prevent a metal ion such as calcium from adsorbed on the contact lens, especially to a soft contact lens, it is desirable to add a chelating agent to the liquid preparation. Examples of the chelating agent include ethylenediamine tetraacetic acid (EDTA) and salts thereof, such as disodium salts of ethylenediamine tetraacetic acid (EDTA• 2Na) and trisodium salts of ethylenediamine tetraacetic acid (EDTA • 3Na). There is adopted the chelating agent generally in a concentration within a range of about 0.01 to about 0.5 w/w % of the liquid preparation for contact lens.

Further, if the pH value of the liquid preparation for a contact lens according to the present invention is excessively high or low, it may cause eye irritation or other problem to the eyes. Therefore, it is generally preferable that the pH

value of the liquid preparation for contact lens is adjusted to be within a range of 4.0 to 9.0, preferably 6.0 to 8.0, and especially around 7.0. For this reason, a suitable pH adjusting agent or buffer may be added, as required.

Examples of the pH adjusting agent, which is used for adjusting the pH, include sodium hydroxide and hydrochloric acid. However, these pH adjusting agents also generate sodium ions and chloride ions, similar to sodium chloride as mentioned above. Therefore, it is needed to restrict the amount of the pH adjusting agent to be used in the liquid preparation to be extremely low. Where the liquid preparation includes ions which constitute strong electrolyte inorganic salts such as sodium chloride due to the addition of the strong alkali or the strong acid to the liquid preparation, it is desirable that the concentration of sodium chloride in the liquid preparation, including sodium chloride formed by the addition of the strong alkali or acid, is not higher than 0.2 w/w %, and preferably not higher than 0.1 w/w %, as mentioned above.

The buffer to effectively keep the pH value of the liquid preparation for contact lens within the above-mentioned range, assuring the safety to the eyes, is suitably selected among conventionally known various buffers. For assuring a high degree of safety to the eyes and reducing the influence on the contact lens, the buffer is preferably selected, for example, from among a citrate buffer, a phosphate buffer, a borate buffer, a carbonate buffer, tris(hydroxymethyl) aminomethane (TRIS) buffer, and a Good-Buffer, such as bis(2-hydroxyethyl)iminotris(hydroxymethyl)methane (Bis-Tris). The buffer is added to the liquid solution in an amount of about 0.01 to about 2 w/w%. Where a buffer, which indicates a relatively strong ionic strength, such as phosphate or citrate, is used, there is an anxiety that the disinfecting effect may be obstructed by the buffer, so that the amount of such buffer needs to be made zero or minimized.

Besides, the present ophthalmic composition may further contain a thickener, as required. Examples of the thickener include: various gums such as heteropolysaccharides; synthetic organic high molecular compounds such as polyvinyl alcohol, poly-N-vinylpyrrolidone, polyethylene glycol, polypropylene glycol, polyacrylamide; cellulose derivatives such as hydroxy ethyl cellulose and hydroxypropyl methylcellulose; and starch derivatives, and these thickeners are advantageously used, as needed.

In preparing the ophthalmic composition of the present invention, which contains the above-mentioned components A to C, the ophthalmic composition can be easily obtained by simply dissolving the respective components in the aqueous medium, such as purified water or distilled water, similar to a preparation of conventional solution, without requiring any special method.

The liquid preparation for a contact lens of the present invention, which is obtained as described above, assures a sufficient safety to the eyes, so that the ophthalmic composition can be preferably used as the contact lens disinfecting solution (disinfectant), a contact lens disinfecting and cleaning solution, a contact lens disinfecting and storing solution, a contact lens disinfecting, cleaning, and storing solution, etc. Moreover, as the liquid preparation for a contact lens according to the present invention assures the safety to the eyes, the liquid preparation can be used as eye drops.

If the contact lens is treated with the liquid preparation according to the present invention, the contact lens, which has been removed from the eye, is placed in a suitable container, which is filled with the liquid preparation of the present invention, for a predetermined period of time, whereby the contact lens is disinfected. When it is needed to wear the contact lens again, the contact lens is taken out of the composition, and then the contact lens is worn. In wearing the contact lens, which has been disinfected as described above, there is only needed to rinse the contact lens with a physiological salt solution. Alternatively, the contact lens can be directly placed on the eye, after the contact lens is taken out of the present liquid preparation, since the present liquid preparation is safe to the eyes.

The types of the contact lenses to be treated with the liquid preparation according to the present invention are not particularly limited. The Examples of the contact lenses can include : soft contact lenses, which are classified into non-water-content contact lenses, low-water-content contact lenses, and high-water-content contact lenses; and hard contact lenses. Considering the properties of the liquid preparation for a contact lens according to the present invention, wherein: the disinfectant (component A) is hard to be attached; the disinfectant has the excellent compatibility with the lens; and the deposition of the crystals and the powdery substances on the contact lens is restricted, the liquid preparation can be especially advantageously adopted for the soft contact lenses, to which the disinfectant and the deposits are easily attached, and of which the sizes are easily changed.

## EXAMPLES

To further clarify the concept of the present invention, some examples of the invention will be described. It is to be understood that the invention is not limited to the details of the illustrated examples and the foregoing description, but may be embodied with various changes, modifications and improvements, which may occur to those skilled in the art without departing from the scope of the invention defined in the attached claims.

Initially, to a sterilized purified water, there were respectively added predetermined additive components according to the ratios as shown in the following TABLE 1, and further added suitable amount of the pH adjusting agent (hydrochloric acid or sodium hydroxide), as needed. Accordingly, there were obtained liquid preparations of the Examples 1 to 19, of which the osmotic pressure was about 290mOsm/kg and the pH was about 7.3.

In the preparation of the liquid preparation for a contact lens, there was used, as the component A, one of the followings: PHMB, which is a germicidal biguanide; and Polyquaternium - 4, Polyquaternium-6, and Polyquaternium-22, which are germicidal quaternary ammonium salts. Besides, as the component B, there was used glycolic acid or asparatic acid, while as the component C, there was used AMPD. In addition, as the neutral amino acid (component D), there was used glycine, while there was used propylene glycol as the nonionic isotonic agent (component E). Moreover, as the nonionic surfactant, there was used HCO-60 (polyoxyethylene (60) hardened castor oil available from Japan Surfactant Kabushiki Kaisha), while there was used, as the chelating agent, EDTA• 2Na. Furthermore, for the reason of comparison, there were respectively used compounds, which have structures and characteristics similar to those of the components B and C, and sodium chloride. As the compounds similar to the component B, there were obtained lactic acid, gluconic acid, and citric acid. As the compounds similar to the component C, there were obtained 2-amino-2-methyl-1-propanol (AMP) and arginine.

The following evaluation tests with respect to the crystals and the powdery substances were implemented for each of thus obtained liquid preparations, and the results were shown in the following TABLE 1.

[Evaluation of the deposition]

Soft contact lenses "Menicon SOFT MA" available from Menicon Co., Ltd. were immersed in each of the above-mentioned liquid preparations for four hours, and this operation was repeated for 30 times. Subsequently, the surfaces of each of the contact lenses were visually observed, and evaluated in accordance with the following evaluation standards. Each of the liquid preparations was replaced with respective new ones, after each one operation of immersing the contact lens.

◎ : Very little change was observed, compared with the surfaces of the lens before the test.

○: Deposition of the crystals and the powdery substances was partially observed, to the extent of giving no problem in using the lens.

X: Deposition was observed on large part of the lens, or large deposits were observed.

[TABLE 1]

| Contents [w/w %] | | Example 1 | 2 | 3 | 4 | 5 | 6 | 7 | 8 | 9 | 10 | 11 | 12 | 13 | 14 | 15 | 16 | 17 | 18 | 19 |
|---|---|---|---|---|---|---|---|---|---|---|---|---|---|---|---|---|---|---|---|---|
| A | PHMB | 1ppm | 1ppm | 1ppm | 1ppm | 1ppm | 1ppm | - | - | - | 1ppm | 1ppm | 1ppm | 1ppm | 1ppm | 1ppm | 1ppm | 1ppm | 1ppm | 1ppm |
| A | Polyquaternium-4 | - | - | - | - | - | - | 100 ppm | - | - | - | - | - | - | - | - | - | - | - | - |
| A | Polyquaternium-6 | - | - | - | - | - | - | - | 100 ppm | - | - | - | - | - | - | - | - | - | - | - |
| A | Polyquaternium-22 | - | - | - | - | - | - | - | - | 100 ppm | - | - | - | - | - | - | - | - | - | - |
| B | Glycolic acid | 0.10 | 0.40 | 1.00 | - | 0.50 | 0.50 | 0.50 | 0.50 | 0.50 | 1.00 | 1.00 | 1.00 | 0.50 | 0.50 | - | - | - | - | - |
| B | Asparatic acid | - | - | - | 0.50 | - | - | - | - | - | - | - | - | - | - | 0.50 | - | - | - | - |
| (B') | Lactic acid | - | - | - | - | - | - | - | - | - | - | - | - | - | - | - | 0.50 | - | - | - |
| (B') | Gluconic acid | - | - | - | - | - | - | - | - | - | - | - | - | - | - | - | - | 0.50 | - | - |
| (B') | Citric acid | - | - | - | - | - | - | - | - | - | - | - | - | - | - | - | - | - | 0.50 | - |
| C | AMPD | 1.65 | 1.60 | 1.45 | 1.60 | 0.73 | 0.73 | 0.73 | 0.73 | 0.73 | 1.00 | 0.70 | 0.20 | - | - | - | 1.60 | 1.60 | 2.00 | - |
| (C') | AMP | - | - | - | - | - | - | - | - | - | - | - | - | 1.40 | - | - | - | - | - | - |
| (C') | Arginine | - | - | - | - | - | - | - | - | - | - | - | - | - | 2.85 | 2.85 | - | - | - | - |
| D | Glycine | - | - | - | - | 0.50 | 0.50 | 0.50 | 0.50 | 0.50 | - | - | - | - | - | - | - | - | - | 0.50 |
| E | Propylene glycol | - | - | - | - | 0.50 | 0.40 | 0.50 | 0.50 | 0.50 | - | - | - | - | - | - | - | - | - | 0.50 |
| | HCO-60 | - | - | - | - | - | 0.05 | 0.05 | 0.05 | 0.05 | - | - | - | - | - | - | - | - | - | 0.05 |
| | EDTA·2Na | - | - | - | - | - | 0.05 | 0.05 | 0.05 | 0.05 | - | - | - | - | - | - | - | - | - | 0.05 |
| | NaCl | - | - | - | - | - | - | - | - | - | - | - | - | - | - | - | - | - | - | 0.43 |
| B:C (molar ratio) | | 1:12 | 1:3 | 1:1 | 1:4 | 1:1 | 1:1 | 1:1 | 1:1 | 1:1 | 1.4:1 | 2:1 | 7:1 | | | | | | | |
| Amount of NaCl in the solution (w/w%) | | 0 | 0 | 0 | 0 | 0 | <0.1 | <0.1 | <0.1 | <0.1 | 0 | 0 | 0 | 0 | 0 | 0 | 0 | 0 | 0 | 0.43 |
| Deposition of crystals | | ○ | ○ | ○ | ○ | ◎ | ◎ | ◎ | ◎ | ◎ | × | × | × | × | × | × | × | × | × | ○ |

(B') and (C') in the above TABLE 1 are similar to the component B and C, respectively, in terms of the structure or the component.

As is apparent from the results shown in the TABLE 1, the evaluations of the deposition of the liquid preparations of the Examples 1 to 9, in which the components A to C are included and the components B and C are combined to fulfill the predetermined molar ratio, are ◎ or ○. From these results, there is understood that the generation of the deposition on the contact lenses is advantageously restricted. In particular, in the Examples 5 to 9, to which the component D and the component E are further added, in addition to the components A to C, the evaluations of the deposition are ◎ , so that an especially excellent effects are exhibited.

On the other hand, although the components of A to C are used in combination in the liquid preparations of the Examples 10 to 12, there are generated depositions on the lenses, because the molar ratio of the component B and the component C is held within 1.4:1 to 7:1. In addition, there are also generated deposits in the liquid preparations of the Examples 13 to 18, in which the compounds similar to the component B or the component C has been used. The evaluation of the deposition of the Example 19, whose osmotic pressure has been adjusted by sodium chloride, is ○.

Besides, the following evaluation tests with respect to the disinfecting effects were implemented for the Examples 1 to 9 and 19, which had been evaluated as ◎ or ○ in the above-mentioned evaluations of the deposition. Thus obtained results were shown in the following TABLE 2.

[Test for examining the disinfecting effect]

9.9mL of each of the solutions was put into respective test tubes. To each of the test tubes, there was added 0.1mL of a fungi liquid, which contained C.a. *(Candida albicans* IFO 1594) as the test bacteria or fungi in an amount of $10^7$ to $10^8$ cfu/mL, so as to provide a fungi suspension including the fungi in an amount of $10^5$ to $10^6$ cfu/mL. The thus obtained specimens were kept at 23°C for four hours. Thereafter, 1mL of each fungi suspension was taken out of each of the test tubes as a sample, and viable cell count per 1mL of each suspension was measured by using 20mL of Glucose Peptone agar medium, by plate dilution method. On the basis of the obtained value, the viable cell count per 1mL of each fungi suspension was calculated. Then, for each of the ophthalmic compositions, an amount of reduction of the fungi was calculated in logarithm (log reduction) according to the following equation:

Log reduction=
log (viable cell count per 1mL of each specimen immediately after inoculation) –
log (viable cell count per 1mL of each fungi suspension after treatment by each liquid preparation for contact lens specimens)

The disinfecting effects were evaluated based on thus obtained values of the log reduction, in accordance with the following evaluation standards.

◎ : The log reduction was not less than 2 (there was a considerable disinfecting effect).
○: The log reduction was not less than 1 and less than 2 (there was a disinfecting effect).
×: The log reduction was less than 1 (the disinfecting effect was weak).

[TABLE 2]

| | Example | | | | | | | | | |
|---|---|---|---|---|---|---|---|---|---|---|
| | 1 | 2 | 3 | 4 | 5 | 6 | 7 | 8 | 9 | 19 |
| Disinfecting effect | ○ | ○ | ◎ | ○ | ◎ | ◎ | ○ | ◎ | ○ | × |
| Log reduction | 1.68 | 1.84 | 2.12 | 1.61 | 2.52 | 2.38 | 1.00 | 2.36 | 1.44 | 0.55 |

As is apparent from the results in the TABLE 2, the disinfecting effects of the liquid preparations of the Examples 1 to 9 are evaluated as ◎ or ○, which explains that the disinfecting effects of the disinfectants are advantageously exhibited. In particular, excellent disinfecting effects can be realized by the liquid preparations of Examples 3, 5, and 6, in which PHMB has been used as the disinfectant and B:C (molar ratio) = 1:1, although the concentration of the disinfectant in the liquid preparation was 1 ppm.

On the other hand, very few disinfecting effect was exhibited by the disinfectant of the Example 19, whose osmotic pressure was adjusted by sodium chloride, although the same amount of the disinfectant is added to the liquid preparation.

## Claims

**1.** A liquid preparation for a contact lens comprising:

(A) at least one disinfectant selected from the group consisting of germicidal biguanides and germicidal quaternary ammonium salts;
(B) glycolic acid and/or asparatic acid; and
(C) 2- amino - 2 - methyl - 1, 3 - propanediol or a salt thereof,

wherein molar ratio of components of said B and C is 1:20 to 1.3:1.

**2.** A liquid preparation for a contact lens according to claims 1, wherein concentration of sodium chloride in the liquid preparation is adjusted to be 0 to 0.2 w/w%.

**3.** A liquid preparation for a contact lens according to claim 1 or 2, wherein component of said A is included in the liquid preparation in a concentration of 0.1 to 500 ppm.

**4.** A liquid preparation for a contact lens according to any one of claims 1 to 3, wherein component of said B is included in the liquid preparation in a concentration of 0.01 to 5 w/w%.

**5.** A liquid preparation for a contact lens according to any one of claims 1 to 4, further containing a neutral amino acid.

**6.** A liquid preparation for a contact lens according to claim 5, wherein said neutral amino acid is included in the liquid preparation in a concentration of 0.1 to 4 w/w%.

**7.** A liquid preparation for a contact lens according to claim 5 or 6, wherein said neutral amino acid is glycine.

**8.** A liquid preparation for a contact lens according to any one of claims 1 to 7, further containing propylene glycol.

**9.** A liquid preparation for a contact lens according to claim 8, wherein concentration of said propylene glycol in the liquid preparation is 0.1 to 1 w/w%.

**10.** A liquid preparation for a contact lens according to any one of claims 1 to 9, further containing at least one of a surfactant and a chelating agent.

**11.** A liquid preparation for a contact lens according to any one of claims 1 to 10, wherein the contact lens to be treated with the preparation is a soft contact lens.

## Patentansprüche

**1.** Flüssiges Präparat für eine Kontaktlinse, umfassend:

(A) zumindest ein aus der aus keimtötenden Biguaniden und keimtötenden quaternären Ammoniumsalzen bestehenden Gruppe ausgewähltes Desinfektionsmittel;
(B) Glycolsäure und/oder Asparaginsäure; und
(C) 2-Amino-2-methyl-1,3-propandiol oder ein Salz davon,

wobei das Molverhältnis der Komponenten von B und C 1:20 bis 1,3:1 beträgt.

2. Flüssiges Präparat für eine Kontaktlinse nach Anspruch 1, worin die Natriumchloridkonzentration im flüssigen Präparat auf 0 bis 0,2 Gew.-% einzustellen ist.

3. Flüssiges Präparat für eine Kontaktlinse nach Anspruch 1 oder 2, worin die Komponente A im flüssigen Präparat in einer Konzentration von 0,1 bis 500 ppm vorliegt.

4. Flüssiges Präparat für eine Kontaktlinse nach einem der Ansprüche 1 bis 3, worin die Komponente B im flüssigen Präparat in einer Konzentration von 0,01 bis 5 Gew.-% vorliegt.

5. Flüssiges Präparat für eine Kontaktlinse nach einem der Ansprüche 1 bis 4, das ferner eine neutrale Aminosäure enthält.

6. Flüssiges Präparat für eine Kontaktlinse nach Anspruch 5, worin die neutrale Aminosäure im flüssigen Präparat in einer Konzentration von 0,1 bis 4 Gew.-% vorliegt.

7. Flüssiges Präparat für eine Kontaktlinse nach Anspruch 5 oder 6, worin die neutrale Aminosäure Glycin ist.

8. Flüssiges Präparat für eine Kontaktlinse nach einem der Ansprüche 1 bis 7, das ferner Propylenglykol enthält.

9. Flüssiges Präparat für eine Kontaktlinse nach Anspruch 8, worin die Propylenglykolkonzentration im flüssigen Präparat 0,1 bis 1 Gew.-% beträgt.

10. Flüssiges Präparat für eine Kontaktlinse nach einem der Ansprüche 1 bis 9, das ferner zumindest eines aus einem Tensid und Chelatbildner enthält.

11. Flüssiges Präparat für eine Kontaktlinse nach einem der Ansprüche 1 bis 10, worin die mit dem Präparat zu behandelnde Kontaktlinse eine weiche Kontaktlinse ist.

## Revendications

1. Préparation liquide pour une lentille de contact comprenant :

   (A) au moins un désinfectant sélectionné dans le groupe consistant en biguanides germicides et sels d'ammonium quaternaire germicides ;
   (B) de l'acide glycolique et/ou de l'acide aspartique; et
   (C) du 2-amino-2-méthyl-1,3-propanediol ou un sel de celui-ci ;

   où le rapport molaire des composants dudit B et C est de 1:20 à 1,3:1.

2. Préparation liquide pour une lentille de contact selon les revendications 1, où la concentration de chlorure de sodium dans la préparation liquide est ajustée pour être comprise entre 0 et 0,2 p/p%.

3. Préparation liquide pour une lentille de contact selon la revendication 1 ou 2, où le composant dudit A est incorporé dans la préparation liquide à une concentration de 0,1 à 500 ppm.

4. Préparation liquide pour une lentille de contact selon l'une quelconque des revendications 1 à 3, où le composant dudit B est incorporé dans la préparation liquide à une concentration de 0,01 à 5 p/p%.

5. Préparation liquide pour une lentille de contact selon l'une quelconque des revendications 1 à 4, contenant de plus un acide aminé.

6. Préparation liquide pour une lentille de contact selon la revendication 5, où ledit acide aminé neutre est incorporé dans la préparation liquide à une concentration de 0,1 à 4 p/p%.

7. Préparation liquide pour une lentille de contact selon la revendication 5 ou 6, où ledit acide aminé neutre est de la

glycine.

8. Préparation liquide pour une lentille de contact selon l'une quelconque des revendications 1 à 7, contenant de plus du propylène glycol.

9. Préparation liquide pour une lentille de contact selon la revendication 8, où la concentration dudit propylène glycol dans la préparation liquide est de 0,1 à 1 p/p%.

10. Préparation liquide pour une lentille de contact selon l'une quelconque des revendications 1 à 9 contenant de plus au moins un agent tensio-actif et un agent complexant.

11. Préparation liquide pour une lentille de contact selon l'une quelconque des revendications 1 à 10, où la lentille de contact à traiter par la préparation est une lentille de contact souple.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10108899 A
- JP 11249087 A
- JP 2001242428 A
- JP 2002136578 A
- JP 2003160482 A
- JP 2000242428 A
- JP 10137327 A
- JP 11052308 A
- JP 2000513001 A
- JP 57132115 A
- JP 2550036 B
- JP 4231054 A
- JP 8512145 A